# EUROPEAN PATENT APPLICATION

(11) **EP 4 484 553 A1**
(43) Date of publication of application: **01.01.2025**
(21) Application number: 23760433.5
(22) Date of filing: 27.02.2023
(51) Int. Cl.: C12N 5/0775, C12N 5/00

(54) **STEM CELL CULTURE METHOD USING SERUM-FREE MEDIUM**

(30) Priority: 25.02.2022 KR 20220025324
(71) Applicant: Kangstem Biotech Co., Ltd., Seoul 06179 (KR)
(72) Inventor: KIM, Mijin, Seoul 08590 (KR); SONG, Seongsoo, Anyang-si, Gyeonggi-do 13934 (KR); AHN, Jongchan, Bucheon-si, Gyeonggi-do 14597 (KR); LEE, Seunghee, Seoul 08797 (KR); HUR, Jihoon, Gwangmyeong-si, Gyeonggi-do 14322 (KR); HEO, Hyunsuk, Gwangmyeong-si, Gyeonggi-do 14322 (KR); KIM, Yeonjeong, Gwangmyeong-si, Gyeonggi-do 14322 (KR); LEE, Mihye, Gwangmyeong-si, Gyeonggi-do 14322 (KR); KANG, Kyungsun, Seoul 06338 (KR)
(74) Representative: Regimbeau
(86) International application number: PCT/KR2023/002742
(87) International publication number: WO 2023/163559

(57) **Abstract**

The present invention relates to a method for enhancing the efficacy of stem cells, comprising a step of culturing stem cells in a serum-free medium.

## Description

### [Technical Field]

The present invention relates to a method for culturing stem cells using a serum-free medium.

### [Background Art]

Stem cells are undifferentiated cells at stages prior to differentiation that are obtainable from embryonic, fetal, and adult tissues and have characteristics of self-renewal capacity, differentiation potency, immortality, *etc.* Stem cells may be classified into pluripotent, multipotent, and unipotent stem cells according to their differentiation potency. Since adult stem cells with multipotency are relatively free from the risk of cancerization, immunorejection, and ethical concerns compared to embryonic stem cells with pluripotency and induced pluripotent stem cells (IPSCs), extensive research thereon and development thereof as therapeutic agents have been in progress. Particularly, in the case of umbilical cord blood-derived mesenchymal stem cells (UCB-MSCs), which are mesenchymal stem cells derived from the blood of newborn babies that circulates through a cardiovascular system of a fetus during pregnancy and is available from a placenta and an umbilical cord after childbirth, after research reports showing that UCB-MSCs have superior proliferation ability to that of stem cells derived from other tissues were published, UCB-MSCs have drawn considerable attention in various fields of study such as the medical field. Particularly, as stem cells separated from discarded tissues, UCB-MSCs have a high value in terms of low donor's burden and recycling of medical waste.

### [Prior Art Document]

(Patent Document 1) Korean Publication Application KR 10-2023-0017786 A

### [Disclosure]

### [Technical Problem]

Accordingly, a method for efficiently culturing stem cells is required.

### [Technical Solution]

One object of the present invention is to provide a method for enhancing the efficacy of stem cells, including a step of culturing stem cells in a serum-free medium.

Another object of the present invention is to provide a method for producing chondrocytes, including the steps of: culturing stem cells in a serum-free medium; and differentiating the cultured stem cells into chondrocytes in a differentiation medium.

Still another object of the present invention is to provide a method for producing a cell therapeutic agent, including the steps of: culturing stem cells in a serum-free medium; and differentiating the cultured stem cells into chondrocytes in a differentiation medium, thereby collecting chondrocytes.

### [Advantageous Effects]

When stem cells are cultured by the method of the present invention, the stem cells can be maintained in a small size, differentiation potency can be increased, and senescence can be delayed or suppressed.

### [Brief Description of Drawings]

FIG. 1 shows the results of measuring the size of umbilical cord blood-derived mesenchymal stem cells by subculturing the same in a conventional serum medium (KSB-3 Complete Medium^{®} Kit) or a serum-free medium (StemVie^{™} XF Medium Kit).
FIG. 2 shows the results of comparing the senescence of stem cells by subculturing umbilical cord blood-derived mesenchymal stem cells in a conventional serum medium (KSB-3 Complete Medium^{®} Kit) or a serum-free medium (StemVie^{™} XF Medium Kit).
FIG. 3 shows the results of comparing the chondrogenic differentiation potency of umbilical cord blood-derived mesenchymal stem cells cultured in a conventional serum medium (KSB-3 Complete Medium^{®} Kit) or a serum-free medium (StemVie^{™} XF Medium Kit).

### [Detailed Description of Preferred Embodiments]

The present invention will be described in detail as follows. Meanwhile, each description and embodiment disclosed herein can be applied to other descriptions and embodiments, respectively. That is, all combinations of various elements disclosed herein fall within the scope of the present invention. Further, the scope of the present invention is not limited by the specific description described below.

Additionally, those of ordinary skill in the art may be able to recognize or confirm, using only conventional experimentation, many equivalents to the particular aspects of the disclosure described herein. Furthermore, it is also intended that these equivalents be included in the present invention.

Additionally, a number of papers and patent documents have been cited throughout the present specification. The content of the cited papers and patent documents is incorporated herein by reference in their entirety and the level of technical field to which the present invention belongs and the contents of the present invention will be described more clearly.

One aspect of the present invention provides a method for enhancing the efficacy of stem cells, including a step of culturing stem cells in a serum-free medium.

As used herein, the serum-free medium refers to a cell culture medium that does not contain or does not substantially contain serum derived from human or non-human animals.

In one embodiment, the serum-free medium may be StemVie^{™} XF Medium Kit.

The StemVie^{™} XF Medium Kit is a commercialized medium available to those skilled in the art. The medium may include glycine, L-arginine, L-cystine · 2HCl, L-glutamic acid, L-histidine, L-isoleucine, L-leucine, L-lysine . HCl, L-methionine, L-phenylalanine, L-serine, L-threonine, L-tryptophan, L-tyrosine . 2Na · 2H₂O, L-valine, choline chloride, D-pantothenic acid (hemiCa), folic acid, myo-inositol, niacinamide, pyridoxine · HCl, riboflavin, thiamine · HCl, magnesium sulfate (anhydrous), potassium chloride, sodium chloride, and D-glucose (dextrose). The medium may further optionally contain inorganic salts, vitamins, buffers, isotonic agents, *etc.*

In the method of the present invention, the enhancement of the efficacy of stem cells may mean any one or more selected from (i) a decrease or maintenance of the size of cells, (ii) an increase in differentiation potency, and (iii) a delay in senescence.

It has been reported that when stem cells are arbitrarily separated based on a certain size, the efficacy of stem cells is increased, *i.e.,* the proliferation ability of small cell groups is relatively high, the expression of senescence-related markers is low, *etc.* In another study, it was confirmed that stem cells with a diameter below a certain size showed a relatively excellent whitening effect, and in still another study, it has been reported that stem cells with high mobility have high differentiation potency and that these cells are relatively small in size. Accordingly, maintaining the cell size relatively small during stem cell culture can lead to enhanced efficacy of stem cells.

In addition, it has been reported that lysosomal proteins, reactive oxygen species (ROS), β-galactosidase activity, *etc.* increase in cells that are aged through stem cell culture. Therefore, in order to measure the degree of senescence, any one or more of lysosomal proteins, reactive oxygen species (ROS), and β-galactosidase activity can be measured. For example, the degree of senescence of stem cells can be determined by measuring β-galactosidase activity.

By culturing stem cells in a serum-free medium according to the present invention, the size of the stem cells may be constantly maintained, and/or (ii) the differentiation potency into desired cells may be increased, and/or (iii) the senescence of the stem cells may be suppressed or delayed, as compared to culturing the stem cells in a serum-containing medium.

By culturing stem cells in a serum-free medium according to the present invention, the size of the stem cells may be constantly maintained, (ii) the differentiation potency into desired cells may be increased, and (iii) the senescence of the stem cells may be suppressed or delayed, as compared to culturing the stem cells in a serum-containing medium.

In any one of the specific embodiments described above, the method of the present invention may be characterized in that (i) the size of the stem cells may be constantly maintained or decreased, and/or (ii) the differentiation potency into desired cells may be increased, and/or (iii) the senescence of the stem cells may be suppressed or delayed, as compared to culturing the stem cells in the KSB-3 Complete Medium^{®} Kit medium.

As used herein, "differentiation" refers to a phenomenon in which the structure or function of a cell is specialized. That is, differentiation is a process in which cells or tissues of organisms are changed into suitable forms and functions for performing tasks individually provided thereto. For example, the term may include not only the differentiation of stem cells into osteocytes, chondrocytes, or fat cells, *etc.,* but also the process by which embryonic stem cells change into ectoderm, mesoderm, and endoderm cells. Additionally, the term also includes an improvement in the ability to differentiate into cells of a particular tissue, when needed.

In one embodiment, the differentiation of the present invention may be the differentiation of stem cells into chondrocytes.

As used herein, "stem cell" refers to a cell capable of differentiating into various tissues, i.e., an undifferentiated cell. The stem cell may be derived from humans or animals, or may be derived from the umbilical cord, umbilical cord blood, bone marrow, fat, muscle, nerve, skin, amnion, or placenta.

In one embodiment, the stem cell of the present invention may be a mesenchymal stem cell.

In any one of the specific embodiments described above, the stem cell of the present invention may be a mesenchymal stem cell derived from umbilical cord blood.

In any one of the specific embodiments described above, the umbilical cord blood-derived mesenchymal stem cell may be an umbilical cord blood-derived pluripotent stem cell that exhibits positive immunological characteristics for ZNF281, a transcriptional regulatory factor, and may show the following characteristics.

In any one of the specific embodiments described above, the umbilical cord blood-derived mesenchymal stem cell may show any one or more characteristics from the following:
(a) shows positive immunological characteristics for c-myc, a transcriptional regulatory factor;
(b) adheres to the extracellular matrix-coated surface and proliferates while forming cell colonies in the form of a line or sphere within 5 to 30 days after adherence;
(c) shows a cumulative population doubling level (CPDL) of 30 to 45;
(d) shows negative immunological characteristics for CD14, CD31, CD34, CD45, and HLA-DR;
(e) is capable of differentiating into mesoderm, endoderm, and ectoderm cells; and
(f) secretes at least one cytokine or chemokine selected from the group consisting of TIMP-2, TGF-β, RANTES CINC-3, EOTAXIN, GM-CSF, IFN-γ, IL-1b, IL-3, IL-6, IL-8, IL-10, IL12p40, IL13, IL-16, IP-10, Leptin, MCP-2, MIG, MIP-3a, b-NGFm, sTNFRI, and PFGF-bb.

In any one of the specific embodiments described above, the umbilical cord blood-derived mesenchymal stem cell may be a cell obtained by separation as disclosed in Korean Patent No. 10-0950195. With respect to the umbilical cord blood-derived mesenchymal stem cells of the present invention, the contents disclosed in Korean Patent Nos. 10-0950195 and 10-1627907 are incorporated by reference.

In one embodiment, the stem cell culture may be a subculture.

As used herein, "subculture" is one of the cell proliferation methods, and is a culture method of transferring cells from a previous culture to a fresh growth medium, for example, every 1 to 7 days. Subculture is a culture method that preserves cell lines and maintains cell generations, and this action is called subculturing or passaging. Through subculture, accumulated toxic metabolites may be removed and depleted nutrients may be supplied, thereby inhibiting cell death and promoting growth and proliferation.

In any one of the specific embodiments described above, subculturing may be performed for two or more passages. In one specific embodiment, subculturing may be performed for 3, 4, or 5 passages, or more.

Another aspect of the present invention provides a method for producing chondrocytes, including the steps of: culturing stem cells in a serum-free medium; and differentiating the cultured stem cells into chondrocytes in a differentiation medium.

Still another aspect of the present invention provides a method for producing a cell therapeutic agent, including the steps of: culturing stem cells in a serum-free medium; and differentiating the cultured stem cells into chondrocytes in a differentiation medium, thereby collecting chondrocytes.

In one embodiment, the serum-free medium may be StemVie^{™} XF Medium Kit.

As used herein, "cell therapeutic agent", which refers to a pharmaceutical product using cells and tissues prepared by isolation from an individual, culture, and specific manipulation and used for the purpose of treatment, diagnosis, and prevention of a disease (FDA regulations, USA), means a pharmaceutical product used for the purpose of treatment, diagnosis, and prevention of a disease through a series of actions such as proliferation and selection of living autologous, allogenic, or xenogenic cells *in vitro,* or changes in biological properties of the cells by different methods, in order to restore the functions of cells or tissues.

The cell therapeutic agent of the present invention may include 1.0×10 cells/ml to 1.0x10⁹ cells/ml, without being limited thereto. The cell therapeutic agent of the present invention may be used in a non-frozen state or may be frozen for subsequent use. In order to freeze the cell therapeutic agent, a standard cryopreservative (e.g. DMSO, glycerol, or Epilife^{™} cell freezing medium (Cascade Biologics)) may be added to the population of cells before freezing. Additionally, the cell therapeutic agent may be administered after being formulated into a unit administration formulation suitable for administration into a patient's body according to a common method in the pharmaceutical field, and the formulation may include an administration dose which is effective after administration once or several times. As formulations suitable for such a purpose, injection agents such as injectable ampoules, injecting agents such as injection bags, and sprays such as aerosol formulations, *etc.* may be used as parenteral formulations. The injectable ampoules may be prepared in a mixed state with injection liquids immediately before use, and as injection liquids, physiological saline solution, glucose, mannitol, Ringer's solution, *etc.* may be used. In addition, the injection bags may be made of polyvinyl chloride or polyethylene, and injection bags from Baxter, Becton-Dickinson, Medcep, National Hospital Products, or Terumo may be used.

The cell therapeutic agent may further include one or more pharmaceutically acceptable common inert carriers, *e.g.,* a preservative, an analgesic, a solubilizer, or a stabilizer for injectable formulations, and a base, an excipient, a lubricant, or a preservative for topical formulations, *etc.*

The cell therapeutic agent of the present invention, prepared as described above, may be administered by any administration method commonly used in the art along with other stem cells, which are used for transplantation and other uses, or in a mixed form with these stem cells, and may be directly engrafted or transplanted to the disease area or directly transplanted or infused into the abdominal cavity of a patient in need of treatment, but is not limited thereto. Furthermore, the administration may be performed either by non-surgical administration using a catheter or by surgical administration via injection or transplantation after incision of the disease area. In addition to parenteral administration according to a common method, for example, direct administration into a lesion, transplantation by infusion into the blood vessel, which is a general method for hematopoietic stem cell transplantation, is also possible.

The cell therapeutic agent may be administered once or as several divided doses. However, it should be understood that the actual dose of active ingredients is determined in consideration of various related factors, such as the disease to be treated, severity of the disease, administration route, a patient's body weight, age, and gender, *etc.,* and thus the administration dose should not be construed as limiting the scope of the present invention in any manner.

### [Mode for Carrying Out the Invention]

Hereinafter, the present invention will be described in detail by way of Examples and Experimental Examples. However, these Examples and Experimental Examples are merely preferred Examples given for illustrative purposes, and thus, the scope of the present invention is not intended to be limited to or by these Examples and Experimental Examples.

### Example 1: Comparative Analysis of the Size of Umbilical Cord Blood-derived Mesenchymal Stem Cells According to Culture Medium Conditions

In order to compare and analyze the cell size of umbilical cord blood-derived mesenchymal stem cells when cultured in a serum-free medium (StemVie^{™} XF Medium Kit) and a conventional serum medium (KSB-3 Complete Medium^{®} Kit), the umbilical cord blood-derived mesenchymal stem cells were seeded at 3,000 cells/ cm² in a 150 mm plate and cultured in a 5% CO₂ incubator at 37°C from P3 to P6. As disclosed in Korean Patent No. 10-0950195, the umbilical cord blood-derived mesenchymal stem cells that showed positive immunological characteristics for ZNF281, a transcriptional regulatory factor, were isolated and used. At each passage time, cells under each culture condition were treated with a TrypLE reagent to obtain single cells.

The obtained single cells were compared and analyzed for cell size using NC200. As a result, the diameters of the cells in each culture condition in P4 were similar, which were found to be 13.7 µm and 13.8 µm for the serum-free medium (StemVie^{™} XF Medium Kit) and the conventional serum medium (KSB-3 Complete Medium^{®} Kit) culture conditions, respectively. However, it was confirmed that in P5, the diameters of the cells were found to be 14 µm and 13.5 µm, respectively, showing a difference of about 0.5 µm, and in P6, the diameters of the cells were found to be 15.4 µm and 13.7 µm, respectively, showing a difference of about 1.7 µm.

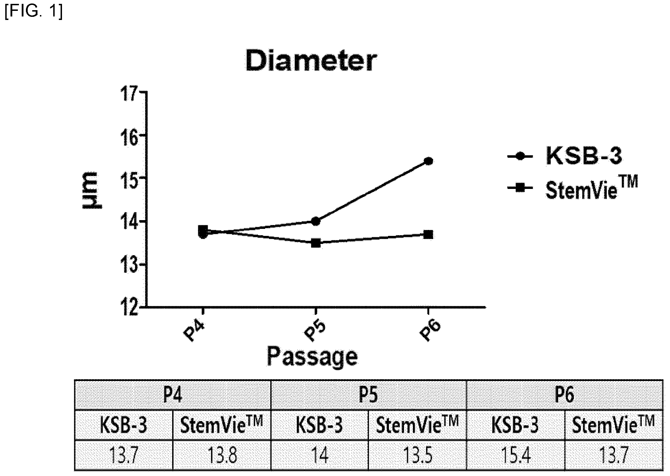

Therefore, it was confirmed that the size of the umbilical cord blood-derived mesenchymal stem cells cultured in the conventional serum medium (KSB-3 Complete Medium^{®} Kit) increased as the passages were continued under the same conditions, whereas the size of the umbilical cord blood-derived mesenchymal stem cells cultured in the serum-free medium (StemVie^{™} XF Medium Kit) did not increase even when the passages were continued under the same conditions (FIG. 1).

### Example 2: Comparative Analysis of Senescence of Umbilical Cord Blood-derived Mesenchymal Stem Cells According to Culture Medium Conditions

In order to compare and analyze the degree of senescence of umbilical cord blood-derived mesenchymal stem cells when cultured in a serum-free medium (StemVie^{™} XF Medium Kit) and a conventional serum medium (KSB-3 Complete Medium^{®} Kit), the degree of senescence of the umbilical cord blood-derived mesenchymal stem cells under each culture condition was compared and analyzed through β-galactosidase staining.

The cells in each culture condition of P4 and P6 obtained in Example 1 above were seeded equally in a 12-well plate at 1×10⁵ cells/mL and cultured in a 5% CO₂ incubator at 37°C for one day. Thereafter, the cells were washed once with PBS and fixed with 4% paraformaldehyde for 10 minutes. After fixation, the cells were washed twice with PBS and treated with 0.5 mL of β-galactosidase staining reagent per well. After staining for 48 hours at 37°C, the cells were examined under a microscope.

As a result of β-galactosidase staining, the SA-β Gal Positive(%) values in P4 were 2.91% and 1.81 % for the serum-free medium (StemVie^{™} XF Medium Kit) and the conventional serum medium (KSB-3 Complete Medium^{®} Kit) culture conditions, respectively, showing that the value for the serum-free medium (StemVie^{™} XF Medium Kit) culture condition was about 1% lower, and in P6, the values were 12.38% and 2.97%, respectively, showing that the value for the serum-free medium (StemVie^{™} XF Medium Kit) culture condition was about 10% lower.

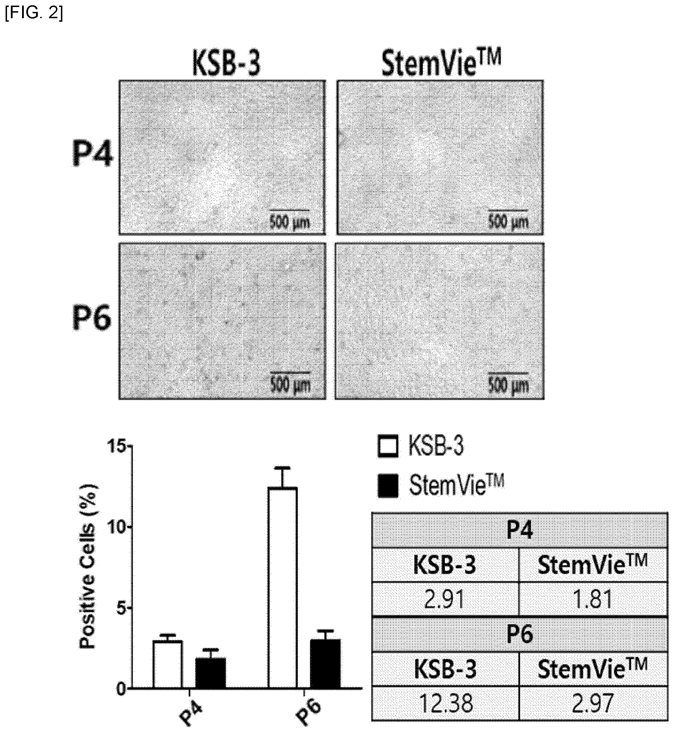

Therefore, it was confirmed that the senescence of the umbilical cord blood-derived mesenchymal stem cells was delayed in the serum-free medium (StemVie^{™} XF Medium Kit) culture conditions when the cells were sub-cultured repeatedly, compared to the conventional serum medium (KSB-3 Complete Medium^{®} Kit) culture conditions (FIG. 2).

### Example 3: Comparative Analysis of Differentiation Potency of Umbilical Cord Blood-derived Mesenchymal Stem Cells According to Culture Medium Conditions

In order to compare and analyze the differentiation potency of umbilical cord blood-derived mesenchymal stem cells when cultured in a serum-free medium (StemVie^{™} XF Medium Kit) and a conventional serum medium (KSB-3 Complete Medium^{®} Kit), chondrogenic differentiation was analyzed in cells cultured under each medium condition.

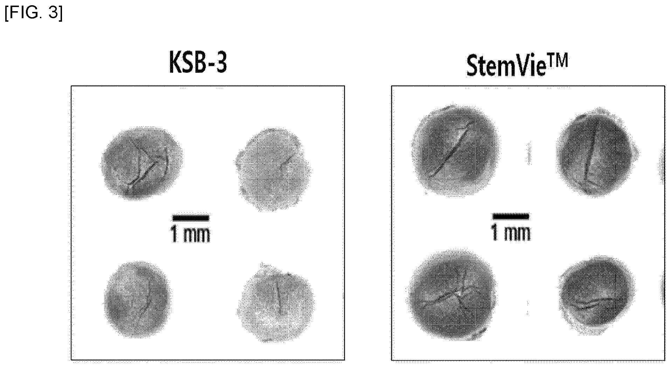

Specifically, the cells in P6 for each culture medium culture condition obtained in Example 1 were dispensed at 3×10⁵ cells/1mL for each 15mL tube, centrifuged, and cultured in a 37°C, 5% CO₂ incubator for one day. The generated pellet was then transferred to a 96-well round plate, and the medium was replaced with a chondrogenic differentiation medium (StemPro^{™} Chondrogenesis Differentiation Kit), and the chondrogenic differentiation medium was replaced three times a week to induce differentiation for three weeks. After differentiation was completed, the pellet was washed with PBS and fixed with 4% PFA, and subjected to Safranin-O staining by commission to a company for analysis. As a result, it was confirmed that chondrogenic differentiation potency was significantly increased when cultured in the serum-free medium (StemVie^{™} XF Medium Kit) compared to the conventional serum medium (KSB-3 Complete Medium^{®} Kit) (FIG. 3).

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A method for enhancing the efficacy of stem cells, comprising a step of culturing stem cells in a serum-free medium,
wherein the serum-free medium is StemVieTM XF Medium Kit, and
the enhancement of the efficacy of stem cells is **characterized by** one or more selected from (i) a decrease or maintenance of the size of cells, (ii) an increase in differentiation potency, and (iii) a delay in senescence.

2. The method of claim 1, wherein the serum-free medium is StemVie^{™} XF Medium Kit, and the method is **characterized by** a decrease or maintenance of the size of stem cells, an increase in differentiation potency, and a delay in senescence, as compared to culturing stem cells in a serum-containing medium.

3. The method of claim 1, wherein the serum-free medium is StemVie^{™} XF Medium Kit, and the method is **characterized by** a decrease or maintenance of the size of stem cells, an increase in differentiation potency, and a delay in senescence, as compared to culturing stem cells in the KSB-3 Complete Medium^{®} Kit medium.

4. The method of claim 1, wherein the differentiation potency is the potency to differentiate into chondrocytes.

5. The method of claim 1, wherein the serum-free medium comprises glycine, L-arginine, L-cystine · 2HCl, L-glutamic acid, L-histidine, L-isoleucine, L-leucine, L-lysine · HCl, L-methionine, L-phenylalanine, L-serine, L-threonine, L-tryptophan, L-tyrosine · 2Na · 2H₂O, L-valine, choline chloride, D-pantothenic acid (hemiCa), folic acid, myo-inositol, niacinamide, pyridoxine · HCl, riboflavin, thiamine · HCl, magnesium sulfate (anhydrous), potassium chloride, sodium chloride, and D-glucose (dextrose).

6. The method of claim 1, wherein the stem cell is a mesenchymal stem cell derived from umbilical cord blood.

7. The method of claim 1, wherein the culture is performed via subculture.

8. The method of claim 7, wherein the subculture is performed for two or more passages.

9. The method of claim 1, wherein the stem cell is an umbilical cord blood-derived pluripotent stem cell that exhibits positive immunological characteristics for ZNF281, a transcriptional regulatory factor, and has the following characteristics:
(a) shows positive immunological characteristics for c-myc, a transcriptional regulatory factor;
(b) adheres to the extracellular matrix-coated surface and proliferates while forming cell colonies in the form of a line or sphere within 5 to 30 days after adherence;
(c) shows a cumulative population doubling level (CPDL) of 30 to 45;
(d) shows negative immunological characteristics for CD14, CD31, CD34, CD45, and HLA-DR;
(e) is capable of differentiating into mesoderm, endoderm, and ectoderm cells; and
(f) secretes at least one cytokine or chemokine selected from the group consisting of TIMP-2, TGF-β, RANTES CINC-3, EOTAXIN, GM-CSF, IFN-γ, IL-1b, IL-3, IL-6, IL-8, IL-10, IL12p40, IL13, IL-16, IP-10, Leptin, MCP-2, MIG, MIP-3a, b-NGFm, sTNFRI, and PFGF-bb.

10. A method for producing chondrocytes, comprising the steps of: culturing stem cells in a serum-free medium; and differentiating the cultured stem cells into chondrocytes in a differentiation medium, wherein the serum-free medium is StemVieTM XF Medium Kit.

11. A method for producing a cell therapeutic agent, comprising the steps of: culturing stem cells in a serum-free medium; and differentiating the cultured stem cells into chondrocytes in a differentiation medium, thereby collecting chondrocytes, wherein the serum-free medium is StemVieTM XF Medium Kit.
